# EUROPÄISCHE PATENTSCHRIFT

(11) **EP 1 313 518 B1**
(45) Veröffentlichungstag und Bekanntmachung des Hinweises auf die Patenterteilung: **23.12.2009**
(21) Anmeldenummer: 01971636.4
(22) Anmeldetag: 28.08.2001
(51) Int. Cl.: A61L 27/44, A61L 27/54, A61L 27/34, A61L 29/12, A61L 29/16, A61L 29/08, A61L 31/12, A61L 31/16, A61L 31/10

(54) **ANTIMIKROBIELLES MATERIAL ZUM IMPLANTIEREN IN KNOCHEN**
ANTIMICROBIAL MATERIAL FOR IMPLANTING IN BONES
MATIERE ANTIMICROBIENNE A IMPLANTER DANS DES OS

(30) Priorität: 31.08.2000 DE 10043151
(43) Veröffentlichungstag der Anmeldung: 28.05.2003
(60) Teilanmeldung: 05023356.8
(73) Patentinhaber: Bio-Gate AG, 90411 Nürnberg (DE)
(72) Erfinder: BECHERT, Thorsten, 96103 Hallstadt (DE); STEINRÜCKE, Peter, 91052 Erlangen (DE)
(74) Vertreter: Gassner, Wolfgang
(86) Internationale Anmeldenummer: PCT/DE2001/003210
(87) Internationale Veröffentlichungsnummer: WO 2002/017984

(56) Entgegenhaltungen:
- EP-A- 0 650 945
- WO-A-84/01721
- WO-A-95/18637
- WO-A-99/26666
- DE-A- 3 110 681
- DE-A- 3 228 849
- US-A- 4 592 920
- US-A- 4 849 223
- US-A- 5 595 750
- US-A- 5 814 272
- US-A- 5 837 275
- US-A- 5 895 419

## Beschreibung

Die Erfindung betrifft ein antimikrobielles Material zun Implantieren in Knochen, zum Beschichten oder Herstellen von Implantaten oder einer implantierbaren Vorrichtung nach dem Oberbegriff des Anspruchs 1.

Aus der EP 0 190 504 ist eine antimikrobielle Zusammensetzung bekannt, welche 5 bis 10 Gew.% Silber enthält. Zur Verbesserung der antimikrobiellen Eigenschaften ist zusätzlich ein hydratisierbares oder ein hydratisiertes Oxid zugesetzt.

Die DE 31 10 681 C2 beschreibt ein Material für Knochenimplantate. Das Material ist aus einem Polymer hergestellt, dem als antimikrobieller Wirkstoff Silberphosphat zugesetzt ist.

Aus der WO 81/02667 ist ein antimikrobielles chirurgisches Implantat bekannt. Dem Implantat ist als antimikrobieller Wirkstoff metallisches Silber zugesetzt.

Die gattungsgemäße WO 82/01990 beschreibt einen Knochenzement auf der Basis von Polymethylmetacrylat als Hauptkomponente, dem als antimikrobieller wirkstoff 5 Vol.% eines Silbersalzes zugesetzt ist.

Die US 5,837,275 offenbart ein antimikrobielles Material, das u.a. Silberpartikel mit einer Korngröße von weniger als 200 nm enthält. Das Silbergitter weist Gitterstörungen und Fehlstellen auf, um die Freisetzung von Silber-Ionen zu erleichtern.

Die US 5,595,750 betrifft ein antimikrobielles Material, bei dem ein aus Bariumsulfat-Partikeln hergestelltes Pulver mit Silber oder einem kupferhaltigen Silber überzogen ist.

Die WO 99/26666 beschreibt eine künstliche Herzklappe, die mit einem Material beschichtet ist, welches antimikrobiell wirkendes Metall enthält.

Aus der WO 84/01721 ist ein mit Silbersulfat oder Silberazetat versehenes Material bekannt. Dieses Material setzt in einer umgebende Flüssigkeit innerhalb von 24 Stunden eine Konzentration von mehr als 1 µM an Silber-Ionen frei.

Die DE 32 288 849 A1 beschreibt ein Material mit einem Überzug aus Silber. Dem Material ist elementarer Kohlenstoff oder Titan zugesetzt. Der Zusatz soll eine erhöhte Freisetzung von Silber-Ionen in die Umgebung erleichtern.

Die US 4,849,233 offenbart einen Knochenzement, dem etwa 10 Gew.% elementares Silber sowie Titanoxid oder Tantaloxid zugesetzt sind. Der Knochenzement zeichnet sich durch eine hohe Rate der Freisetzung an Silber-Ionen aus.

Die antimikrobielle Wirksamkeit der nach dem Stand der Technik bekannten Materialien ist mit der sogenannten Hemmhofmessung nachgewiesen worden. Die Hemmhofmessung ist z.B. beschrieben in Raad I. et al . , J. Infec. Dis. 173 (1996). Dabei wird das zu prüfende Material in ein Nährmedium, z.B. Agar, eingebettet. Wegen der Freisetzung antimikrobiell wirkender Metall-Ionen bildet sich um das Material ein Hemmhof. Die Ausbildung und die Größe eines solchen Hemmhofs ist nach dem Stand der Technik als Anzeichen für die antimikrobielle Wirksamkeit des Materials gewertet worden. Die nach dem Stand der Technik bekannten Materialien haben z.T. den Nachteil, daß sie nur für eine relativ kurze Zeit eine ausreichend hohe Konzentration an Silber-Ionen freisetzen. Deren antimikrobielle Wirksamkeit ist auf diese Zeit beschränkt. Um diesem Nachteil entgegenzuwirken, setzt man nach dem Stand der Technik relativ hohe Mengen an antimikrobiell wirkenden Metallen zu. Das wiederum führt in vivo zu unerwünschten zelltoxischen Effekten.

Aufgabe der Erfindung ist es, die Nachteile nach dem Stand der Technik zu beseitigen. Es soll insbesondere ein möglichst einfach und kostengünstig herstellbares antimikrobielles Material mit verbesserten Eigenschaften angegeben werden. Das Material soll für den Patienten möglichst verträglich sein. Weiterhin soll ein Implantat angegeben werden.

Diese Aufgabe wird durch die Merkmale der Ansprüche 1 und 10 gelöst. Zweckmäßige Ausgestaltungen ergeben sich aus den Merkmalen der ansprüche 2 bis 9.

Nach Maßgabe der Erfindung ist u. a. vorgesehen, daß das Metall aus Aggregaten von Primärpartikeln mit einer mittleren Korngröße zwischen 10 und 100 nm gebildet ist.

Bei den erfindungsgemäßen Aggregaten lassen sich die Primärpartikel aufgrund ihrer äußeren Form noch identifizieren. Die Primärpartikel sind miteinander im wesentlichen über Sinterhälse gebunden. Die Aggregate bilden eine hochporöse Gerüststruktur. Das Matrixmaterial kann in Abhängigkeit der jeweiligen Ausgestaltung im wesentlichen bioinert sein.

Das vorgeschlagene Material zeichnet sich durch eine hervorragende antimikrobielle wirksamkeit aus. An der Oberfläche des Materials wird eine ausreichend hohe Konzentration an Silber-Ionen zur Verfügung gestellt. Dabei ist die Rate der Diffusion von Silber-Ionen in das umgebende Gewebe besonders gering. Damit bleibt die antimikrobielle Wirksamkeit auf die Oberfläche des Materials beschränkt. Mit dem erfindungsgemäβen Material können z.B. Knochenzemente, Implantate oder auch implantierbare Vorrichtungen, wie Katheter, mit verbesserten antimikrobiellen Eigenschaften hergestellt werden. Es treten keine unerwünschten zytotoxischen Effekte auf. Die antimikrobielle Wirkung des Materials ist besonders lang anhaltend. Der Patient wird pharmakologisch weniger belastet.

Die Aggregate weise eine mittlere Korngröße von 10 bis 20 µm auf. Die Oberfläche der Aggregate beträgt zweckmäßigerweise 3 bis 6 m²/g. Sie können eine Porosität von bis zu 95% aufweisen. Zweckmäßigerweise liegt die Porosität zwischen 70 und 95%. Die vorgenannten Merkmale tragen zu einer gleichmäßigen und zytotoxisch unbedenklichen Abgabe von Silber-Ionen an der Oberfläche des Materials bei.

Die Aggregate können mittels Inertgasverdampfung und Kondensation, vorzugsweise bei einem Inertgasdruck, von 10 bis 100 mbar, hergestellt werden. Das Metall kann aus einem oder mehreren der folgenden Bestandteile gebildet sein: Ag, Au, Pt, Pd, Ir, Sn, Cu, Sb, Zn. Das Metall weist zweckmäßigerweise einen im wesentlichen ungestörten Gitteraufbau auf. So wird eine unerwünscht hohe Freisetzung von Silber-Ionen in das umgebende Gewebe vermieden.

Nach einem besonders vorteilhaften Ausgestaltungsmerkmal sind höchstens 2 Gew.%, vorzugsweise 0,01 bis 2 Gew.%, an Metall bezogen auf das Gewicht des Matrixmaterial enthalten. Zweckmäßigerweise findet als Metall Silber Verwendung. Der vorgeschlagene Zusatz an Metall ist relativ gering. Das Material kann preisgünstig hergestellt werden.

Es hat sich weiter als zweckmäßig erwiesen, daß die Aggregate mit dem Matrixmaterial vollständig infiltriert sind. Die Aggregate sind vorteilhafterweise homogen im Matrixmaterial dispergiert bzw. verteilt. Diese Merkmale tragen dazu bei, daß an allen Orten der Oberfläche des Materials stets eine gleich große Menge an Silber-Ionen freigesetzt wird.

Bei dem Matrixmaterial kann es sich um ein, vorzugsweise aus mehreren Komponenten gebildetes, Polymer handeln. Das Polymer kann im wesentlichen Acrylsäure- und/oder Metacrylsäureester enthalten. Als Matrixmaterial eignen sich aber auch andere nach dem Stand der Technik zur Herstellung von Knochenzementen verwendete Matrixmaterialien.

Das vorgeschlagene antimikrobielle Material eignet sich zur Herstellung oder auch zur Beschichtung von Implantaten oder einer implantierbaren medizinischen Vorrichtung, z.B. ein Katheter oder Intratachealtuben. Insbesondere können, z.B. aus Titan oder Keramik hergestellte Hüftgelenksimplantate, Herzklappen, stents, Kniegelenksimplantate, Zahnfüllungen, Kontaktlinsen oder Intraokularlinsen mit dem vorgeschlagenen antimikrobiellen Material beschichtet oder hergestellt werden.

Zur Herstellung des erfindungsgemäßen Materials wird ferner ein Verfahren mit folgenden Schritten vorgeschlagen:
a) Verdampfen und Kondensieren von Metall unter Inertgasatmosphäre, wobei der Druck des Inertgases und die Verdampfungstemperatur so eingestellt werden, daß aus Primärpartikeln mit einer mittleren Korngröße von 10 bis 100 nm bestehende Aggregate sich bilden und
b) Mischen der Aggregate mit einem aushärtbaren Matrixmaterial.

Das vorgeschlagene Verfahren läßt sich relativ einfach durchführen. Es kann damit das antimikrobielle Material in gleichbleibender Qualität und relativ preisgünstig hergestellt werden.

Nach einem Ausgestaltungsmerkmal werden die Aggregate nach dem Schritt lit. a klassiert. Zweckmäßigerweise wird eine Korngrößenfraktion der Aggregate im Bereich von 1 bis 20 µm, vorzugsweise 10 bis 20 µm, mit dem, vorzugsweise im flüssigen Zustand vorliegenden, Matrixmaterial gemischt. Die Korngrößenfraktion kann in das Matrixmaterial eingerührt werden.

Es hat sich als zweckmäßig erwiesen, ein Inertgas zu verwenden, das als einen wesentlichen Bestandteil mindestens eines der folgenden Gase enthält: Argon, Krypton, Xenon, Helium.

Wegen der weiteren vorteilhaften Ausgestaltungen wird auf die vorangegangenen Ausführungen verwiesen. Die dort beschriebenen Merkmale können sinngemäß auch beim Verfahren Anwendung finden.

Nachfolgend werden Ausführungsbeispiele der Erfindung anhand der Zeichnung näher erläutert. Es zeigen:
- Fig. 1: die bakterielle Proliferation an Knochenzementen im Vergleich zwischen Silberpulvern nach dem Stand der Technik und dem erfindungsgemäßen Silberpulver,
- Fig. 2: eine rasterelektronenmikroskopische Aufnahme eines Silberaggregats,
- Fig. 3: die Abhängigkeit der Zytotoxizität eines Knochenzements in Anhängigkeit des Gehalts an Silber und
- Fig. 4: einen Hemmhoftest für verschiedene Knochenzemente.

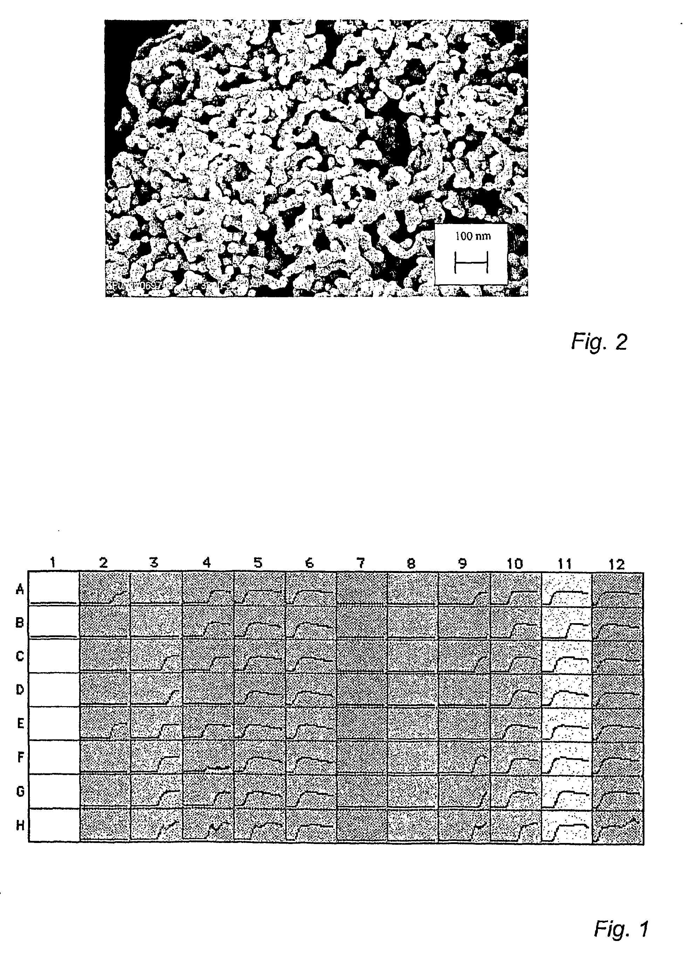

Die in den Fig. 1 gezeigten Ergebnisse sind nach dem aus der DE 197 51 581 A1 bekannten Verfahren ermittelt worden. Dieses Verfahren ist ferner beschrieben in Bechert, Thorsten et al., Nature Medicine, Vol. 6, No. 8 (09/2000). Der Offenbarungsgehalt der beiden vorgenannten Dokumente wird hiermit einbezogen.

Es werden zunächst jeweils 8 Parallelproben (A - H) derselben Charge an Knochenzement angefertigt. Die Proben sind üblicherweise zylinderförmig ausgebildet. Sie weisen eine Länge von etwa 1 cm und einen Durchmesser von 2 bis 5 mm auf. Anschließend wird in jede Vertiefung der Mikrotiterplatte 200 µl einer Bakterien-enthaltenden Lösung gefüllt. Die Proben werden bei 37°C für eine Stunde inkubiert. Die Proben werden dann entnommen und dreimal mit physiologischem Puffern gewaschen. Anschließend werden die Proben in die Vertiefungen einer Mikrotiterplatte gelegt, welche mit einem Minimalmedium gefüllt sind. Pro Vertiefung werden 200 µl an Minimalmedium eingefüllt. Die Proben werden für 24 Stunden bei 37°C inkubiert. Anschließend werden die Proben entnommen und verworfen. Zu jeder Vertiefung der Mikrotiterplatte werden 50 µl eines Vollmediums (Trypcasesoja) zugegeben. Anschließend wird die Trübung der Lösung im Abstand von 30 Minuten über einen Zeitraum von 48 Stunden gemessen. Die Lösung wird dabei auf einer Temperatur von 37°C gehalten. Die Trübungsmessung erfolgt mit Licht einer Wellenlänge von 578 nm mittels eines geeigneten Lesegeräts. Eine Trübung zeigt an, daß Bakterien von der Oberfläche der Probe in die Umgebung abgegeben worden sind.

Fig. 1 zeigt einen Vergleich eines Knochenzements, dem unterschiedliche Gehalte an herkömmlichem Silberpulver der Firma Chempur (Spalten 2 - 6) zugesetzt worden sind, mit einem zweiten Knochenzement, dem vergleichbare Mengen an erfindungsgemäßen Silberaggregaten zugesetzt worden sind (Spalten 7 bis 11).

Den Proben der Spalten 2 und 7 sind 2,0 Gew.% Silber, den Proben der Spalten 3 und 8 1,0 Gew.% Silber, den Proben der Spalten 4 und 9 0,5 Gew.% Silber, den Proben der Spalten 5 und 10 0,1 Gew.% Silber und den Proben der Spalten 6 und 11 0,05 Gew.% Silber zugesetzt worden. Spalte 12 gibt die Ergebnisse von Proben ohne Silberzusatz (Kontrolle) wieder.

Es zeigt sich, daß bereits ein Zusatz von 1,0 Gew.% an erfindungsgemäßen Silberaggregaten eine ausgezeichnete antimikrobielle Wirksamkeit zur Folge hat. Bei der Verwendung herkömmlicher Silberpulver wird selbst bei einem Zusatz von 2,0 Gew.% keine zuverlässige antimikrobielle Wirksamkeit erreicht.

Fig. 2 zeigt eine rasterelektronenmikroskopische Aufnahme des erfindungsgemäßen Silberaggregats. Das Silberaggregat besteht im wesentlichen aus kugeligen Primärpartikeln mit einer mittleren Korngröße von etwa 20 nm. Die Primärpartikel sind im wesentlichen über Sinterhälse miteinander verbunden. Sie bilden ein hochporöses Gerüst. Das hier gezeigte Silberaggregat hat eine Größe von etwa 10 µm.

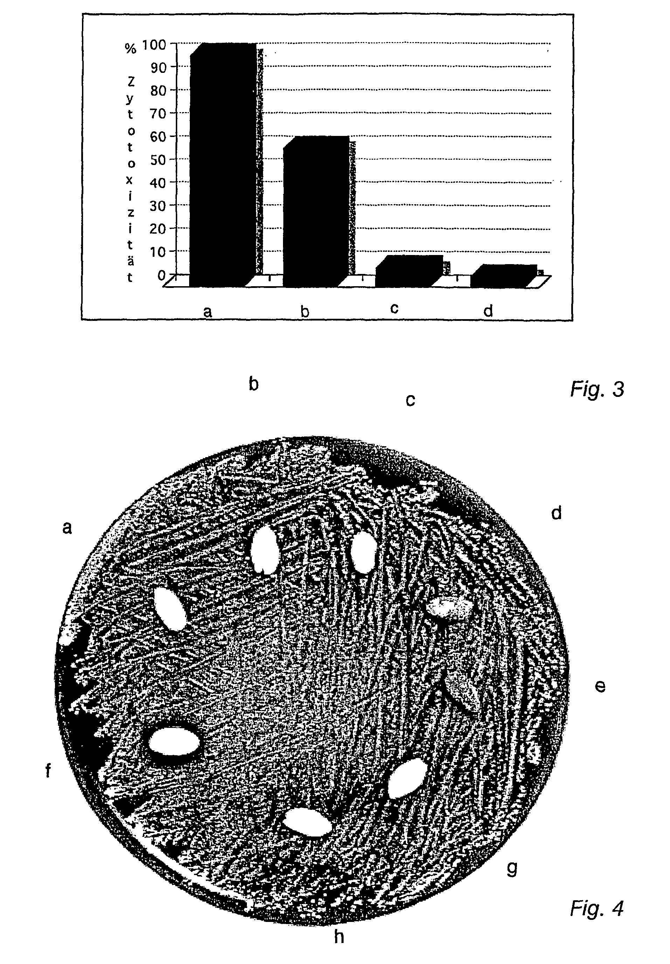

Fig. 3 zeigt die Ergebnisse der zelltoxischen Wirkung der erfindungsgemäßen Knochenzemente. Als Verfahren diente hier der Test nach Greil et al. (Infection, Vol. 27, 1999, Suppl. 1, S. 34 - 37). Dabei wird ein Tetrazolfarbstoff (MTT) durch eine atmungsaktive vitale Zellinie (MRC-5 Zellen oder durch Polyhämagglutinin stimulierte Lymphozyten) in ein intensiv gefärbtes Formazan umgewandelt. Das Ausmaß der in einem vorgegeben Zeitabschnitt erzielten Verfärbung ist ein Maß für die Vitalität der Zellen. Die Durchführung des Tests erfolgte nach der ISO-Richtlinie. Zunächst werden dazu Extrakte des Knochenzements mit Kulturmedium über 24 Stunden bei 37°C Proben gewonnen. Die Proben werden im Formazan-Assay zusammen mit den Zellen für eine Dauer von 72 Stunden inkubiert. Die Zytotoxizität definiert sich als der in Folge der Extraktzugabe durch Formazanbildung definierte relative prozentuale Verlust der Atmungsaktivität.

Als Positivkontrolle dienten in unabhängigen Experimenten aus PVC entsprechend der ISO-Richtlinie gewonnen Extrakte. Zytotoxizitätswerte größer oder gleich 30% werden als zelltoxische Wirkungen gewertet. Als Kontrollen dienten Extrakte von PE (Negativkontrolle) bzw. PVC (Positivkontrolle).

Fig. 3a zeigt das Ergebnis eines 1:10 verdünnten Extrakts für die Positivkontrolle mit Lymphozyten. Die Zytotoxizität beträgt hier 100%.

Fig. 3b zeigt das Ergebnis der Positivkontrolle mit MRC-5 Zellen. Die Zytotoxizität beträgt hier 60%.

Fig. 3c zeigt das Ergebnis unter Verwendung eines erfindungsgemäßen Knochenzements mit 1,0 Gew.% Silberaggregatzusatz. Die Zytotoxizität betrug hier lediglich 8,4% für Lymphozyten und 4,8% für MRC-5 Zellen (Fig. 3d).

Fig. 4 zeigt die Ergebnisse einer Hemmhofmessung von erfindungsgemäßen Knochenzementen im Vergleich zu herkömmlichen Knochenzementen. Die Probenzusammensetzung war wie folgt:
- Probe lit. a:: Knochenzement mit 0,05 Gew.% an Silberaggregaten,
- Probe lit. b:: Knochenzement mit 0,1 Gew.% an Silberaggregaten,
- Probe lit. c:: Knochenzement mit 0,5 Gew.% an Silberaggregaten,
- Probe lit. d:: Knochenzement mit 2,0 Gew.% an Silberaggregaten,
- Probe lit. e:: Knochenzement mit 5,0 Gew.% an Silberaggregaten,
- Probe lit. f:: herkömmlicher Gentamycin-haltiger Knochenzement (Firma Merck, "Palacos")
- Probe lit. g, h:: herkömmliche Knochenzemente ohne Zusätze (Firma Merck, "Palacos").

Die Proben lit. a bis lit. h sind zur Durchführung der Hemmhofmessung in einen Müller-Hinton-Agar eingebettet worden, der mit Koagulase-negativen Staphylokken als Testkeim für 24 Stunden bebrütet worden ist. Bei den Silberaggregat-haltigen Knochenzementen ist kein Hemmhof erkennbar. Der herkömmliche Gentamycin-haltige Knochenzement zeigt dagegen einen deutlichen Hemmhof. Die erfindungsgemäßen Knochenzemente setzen also nur eine geringe Konzentration an Silber-Ionen in die Umgebung frei.

## Patentansprüche

1. Antimikrobielles Material zum Implantieren in Knochen, zum Beschichten oder Herstellen eines Implantats oder einer implantierbaren medizinischen Vorrichtung, wobei aus einem antimikrobiellen Metall gebildete Aggregate in einem, im ausgehärteten Zustand eine Matrix bildenden Matrixmaterial fein verteilt sind,
**dadurch gekennzeichnet, dass**
die Aggregate (eine mittlere Korngröße von 10 bis 20 µm und eine hochporöse Gerüststruktur aufweisen,
und dass die Gerüststruktur aus über Sinterhälse miteinander verbundenen, in ihrer Form noch identifizierbaren Primärpartikeln mit einer mittleren Korngröße zwischen 10 und 100 nm gebildet ist.

2. Antimikrobielles Material nach Anspruch 1, wobei die Aggregate eine Oberfläche von 3 bis 6 m² pro Gramm aufweisen.

3. Antimikrobielles Material nach einem der vorhergehenden Ansprüche, wobei die Aggregate eine Porosität von 70 bis 95% aufweisen.

4. Antimikrobielles Material nach einem der vorhergehenden Ansprüche, wobei das Metall aus einem oder mehreren der Eolgenden Bestandteile gebildet ist: Ag, Au, Pt, Pd, Ir, Sn, Cu, Sb, Zn.

5. Antimikrobielles Material nach einem der vorhergehenden Ansprüche, wobei höchstens 2,0 Gew.%, vorzugsweise 0,01 bis 2,0 Gew.%, an Metall bezogen auf das Gewicht des Matrixmaterials enthalten sind.

6. Antimikrobielles Material nach einem der vorhergehenden Ansprüche, wobei das Polymer Acylsäure- und/oder Metacrylsäureester enthält.

7. Antimikrobielles Material nach einem der vorhergehenden Ansprüche, wobei die Aggregate mit dem Matrixmaterial vollständig infiltriert sind.

8. Antinikrobielles Material nach einem der vorhergehenden Ansprüche, wobei das Matrixmaterial ein, vorzugsweise aus mehreren Komponenten gebildetes, Polymer ist.

9. Antimikrobielles Material nach einem der vorhergehenden Ansprüche, wobei die Aggregate homogen im Implantatwerkstoff dispergiert sind.

10. Implantat oder implantierbare medizinische Vorrichtung gebildet aus oder zumindest abschnittsweise beschichtet mit dem antimikrobiellen Material nach einem der vorhergehenden Ansprüche.

## Claims

1. An antimicrobial material for implanting in bones, for coating or producing an implant or an implantable medical device, wherein aggregates formed of an antimicrobial metal are finely dispersed in a matrix material which forms a matrix in the cured state,
**characterized in that**
the aggregates have an average particle size of 10 to 20 µm and a highly porous framework structure,
and that the framework structure is formed of primary particles having an average particle size between 10 and 100 nm, wherein the primary particles are bound together by necks formed during sintering, wherein the shape of the primary particles can still be identified.

2. The antimicrobial material as claimed in claim 1, wherein the aggregates have a surface area of 3 to 6 m² per gram.

3. The antimicrobial material as claimed in any of the preceding claims, wherein the aggregates have a porosity of 70% to 95%.

4. The antimicrobial material as claimed in any of the preceding claims, wherein the metal is formed of one or more of the following constituent parts: Ag, Au, Pt, Pd, Ir, Sn, Cu, Sb, Zn.

5. The antimicrobial material as claimed in any of the preceding claims, wherein the metal content is not more than 2.0% by weight, preferably 0.01 to 2.0% by weight, based on the weight of the matrix material.

6. The antimicrobial material as claimed in any of the preceding claims, wherein the polymer comprises acylic esters and/or metacrylic esters.

7. The antimicrobial material as claimed in any of the preceding claims, wherein the aggregates are completely infiltrated with the matrix material.

8. The antimicrobial material as claimed in any of the preceding claims, wherein the matrix material is a polymer which is preferably formed of a plurality of components.

9. The antimicrobial material as claimed in any of the preceding claims, wherein the aggregates are homogeneously dispersed in the implant material.

10. An implant or implantable medical device formed of or at least partially coated with the antimicrobial material as claimed in any of the preceding claims.

## Revendications

1. Matériau antimicrobien pour l'implantation dans l'os, pour le revêtement ou la fabrication d'un implant ou d'un dispositif médical implantable, où des agrégats réalisés en un métal antimicrobien sont répartis finement en un matériau à matrice constituant une matrice à l'état trempé,
**caractérisé en ce que**
les agrégats présentent une taille de grains moyenne comprise entre 10 et 20 µm et une structure de texture hautement poreuse,
et que la structure de texture est composée de particules primaires encore identifiables dans leur forme d'une taille de grains moyenne entre 10 et 100 nm reliées entre elles par l'intermédiaire de pontages frittés.

2. Matériau antimicrobien selon la revendication 1, où les agrégats présentent une surface comprise entre 3 et 6 m² par gramme.

3. Matériau antimicrobien selon l'une des revendications précédentes, où les agrégats présentent une porosité de 70 à 95 %.

4. Matériau antimicrobien selon l'une des revendications précédentes, où le métal est constitué en un ou plusieurs des éléments suivants : Ag, Au, Pt, Pd, Ir, Sn, Cu, Sb, Zn.

5. Matériau antimicrobien selon l'une des revendications précédentes, où sont contenus au maximum 2,0 % en poids, de préférence entre 0,01 et 2,0 % en poids, de métal par rapport au poids du matériau à matrice.

6. Matériau antimicrobien selon l'une des revendications précédentes, où le polymère contient de l'ester acrylique et/ou de l'ester méthacrylique.

7. Matériau antimicrobien selon l'une des revendications précédentes, où les agrégats sont entièrement infiltrés avec le matériau à matrice.

8. Matériau antimicrobien selon l'une des revendications précédentes, où le matériau à matrice est un polymère, formé de préférence de plusieurs composants.

9. Matériau antimicrobien selon l'une des revendications précédentes, où les agrégats sont dispersés de manière homogène dans le matériau d'implant.

10. Implant ou dispositif médical implantable composé ou au moins partiellement revêtu d'un matériau antimicrobien selon l'une des revendications précédentes.
